# EUROPEAN PATENT APPLICATION

(11) **EP 2 578 211 A1**
(43) Date of publication of application: **10.04.2013**
(21) Application number: 10852593.2
(22) Date of filing: 24.12.2010
(51) Int. Cl.: A61K 31/122, A61K 9/107, A61K 47/38, A61P 43/00, A61P 39/06

(54) **6-DECAPRENYL-2,3-DIMETHOXY-5-METHYL-1,4-BENZOQUINONE-BASED COMPOSITION AND METHOD FOR THE PRODUCTION THEREOF**

(30) Priority: 02.06.2010 RU 2010122471
(71) Applicant: Obschestvo S Ogranichennoi Otvetstvennostju "Accent", Moscow 123056 (RU)
(72) Inventor: ENILEEV, Rustam Khalilovich, Moscow 123458 (RU); SHABATIN, Vladimir Petrovich, Moscow 117342 (RU)
(74) Representative: Cottrill, Emily Elizabeth Helen
(86) International application number: PCT/RU2010/000783
(87) International publication number: WO 2011/152751

(57) **Abstract**

The invention relates to the pharmaceutical industry, and specifically to a composition based on 6-decaprenyl-2,3-dimethoxy-5-methyl-1,4-benzoquinone (ubinone, ubiquinone, coenzyme Q10) in liquid form, which can be used for the prophylaxis and treatment of various illnesses, for restoring fitness for work, and also as a food additive. In addition to the active substance, the composition comprises a nonionic surface-active substance, an antioxidant, a preservative, a fat-soluble emulsion stabilizer, water-soluble cellulose derivatives, a water-soluble emulsion stabilizer and water. The composition is characterized by high bioaccessibility and an extended storage life. The method for producing the composition consists in that the nonionic surface-active substance and the antioxidant are mixed and heated to 40-120°C, the fat-soluble emulsion stabilizer and 6-decaprenyl-2,3-dimethoxy-5-methyl-1,4-benzoquinone are dissolved in the solution produced, the resultant mixture is cooled to 30-60°C and poured under intensive stirring into a mixed solution, preheated to 30-100°C, of the water-soluble cellulose derivatives, water-soluble emulsion stabilizer and preservative in water.

## Description

The invention relates to the pharmaceutical industry and can be used in medicine for prophylaxis and treatment of different diseases, restoration of efficiency. It also can be used as a food supplement.

6-decaprenyl-2,3-dimethoxy-5-methyl-1,4-benzoquinone (ubinone, ubiquinone, coenzyme Q10) is known to be used in prophylaxis and treatment of several diseases of cardiovascular and nerve system as well as of diseases, associated with disorders of mitochondrial energetics and production of free radicals. Particularly it is used in the therapy of coronary heart disease, heart failure, atherosclerosis, myocardial infarction, hypertension, hypercholesterolemia, cardiomyopathy, diabetes, periodontal diseases, chronic fatigue syndrome and asthenia, migraine, age-related macular degeneration, asthenozoospermia, Friedreich's ataxia, Parkinson's disease, obesity, for protection of myocardium during surgical operations and for restoration of efficiency (Littarru GP, Tiano L. Clinical aspects of coenzyme Q10: an update. Curr Opin Clin Nutr Metab Care. 2005 Nov; 8(6):641-6).

6-decaprenyl-2,3-dimethoxy-5-methyl-1,4-benzoquinone is a crystalline powder with yellow-orange color, odorless, water-insoluble (Temporal pharmacopoeial monograph 42 - 1978 - 90). Due to its hydrophobicity this substance is practically not assimilated by an organism. Thereby in a solid form it is not used in therapy and prophylaxis of different diseases. To improve its bioavailability ubiquinone must be dissolved in lipophilic media or nonionic surface-active substances.

There is a composition based on 6-decaprenyl-2,3-dimethoxy-5-methyl-1,4-benzoquinone (ubiquinone or ubinone), containing 7,5 wt.% ubinone in terms of 100% substance, 0,0135 wt.% butylhydroxytoluene and 92,4865 macc.% refined sunflower oil (Mashkovsky M. Pharmaceuticals. 12th edition, Part 2, Moscow: Medicine, 1993, p. 217. Temporal pharmacopoeial monograph 42 - 1851 - 88 "Ubinone capsules 0,015 g").

Refined sunflower oil in this composition is used to dissolve the active substance. Butylhydroxytoluene acts in this composition as an antioxidant - a related substance. But it is known that bioavailability of an active substance in oil solutions is worse than in water solutions or water-oil emulsions.

There is a composition, containing the active substance - 6-decaprenyl-2,3-dimethoxy-5-methyl-1,4-benzoquinone, nonionic surface-active substance, antioxidant, preservative, liposoluble or/and water soluble emulsion stabilizer and powdery agent. The method of preparation of this composition is the following: the mixture of dimethoxy-5-methyl-1,4-benzoquinone, nonionic surface-active substance, antioxidant, preservative, liposoluble emulsion stabilizer, water soluble emulsion stabilizer and water is heated to 30 - 120°C and dispersed by mixing in a powdery agent. (Application Nº 2008128728 for Patent RF on 16.07.2008).

The invention ensures production of a powder composition, stable at storage during 39 months. The time of appearance of the active substance - 6-decaprenyl-2,3-dimethoxy-5-methyl-1,4-benzoquinone in blood of rabbits at oral administration is (19± 4) minutes.

This powder composition is used in the pharmaceutical industry in a form of tablets (Specification 9197-039-58693373-07 "Biologically active food supplement Qudesan Forte"). Such preparative form allows to increase shelf life of the composition but at the same time it reduces bioavailability of the composition because of extra time, required for dissolution of a tablet.

The composition based on 6-decaprenyl-2,3-dimethoxy-5-methyl-1,4-benzoquinone and the method of its preparation are the most similar in technical essence and the achieved result (Patent RF Nº 2290176 on 01.12.2005). The composition contains the active substance, nonionic surface-active substance, antioxidant, preservative, liposoluble emulsion stabilizer, water soluble emulsion stabilizer and water. The method of preparation is the following: certain amount of nonionic surface-active substance and antioxidant are mixed, heated to 40 - 120°C, the required amount of liposoluble emulsion stabilizer and 6-decaprenyl-2,3-dimethoxy-5-methyl-1,4-benzoquinone are dissolved in the resulted solution, and this mixture is added by intensive mixing to the dissolved in water preheated to 30 - 100°C mixture of water soluble emulsion stabilizer and preservative.

As the result a water-oil emulsion stable for 2 years is obtained with improved bioavailability in comparison to an oil solution of ubiquinone.

The shortcoming of this composition is that during long-term storage (over 25 months) the water-oil emulsion separates. It leads to loss of its pharmaceutical properties.

The aim of this invention is to produce a composition based on 6-decaprenyl-2,3-dimethoxy-5-methyl-1,4-benzoquinone in a liquid form with prolonged shelf life for prophylaxis and treatment of different diseases and/or restoration of efficiency.

The technical result consists in increase stability and prolonged shelf life of the water-oil emulsion and at the same time maintenance of high bioavailability of active substances.

The proposed composition contains 6-decaprenyl-2,3-dimethoxy-5-methyl-1,4-benzoquinone, nonionic surface-active substance, antioxidant, preservative, liposoluble emulsion stabilizer, water soluble cellulose derivatives, water soluble emulsion stabilizer and water with the following proportion of components, wt.%:

| | |
|---|---|
| 6-decaprenyl-2,3-dimethoxy-5-methyl-1,4-benzoquinone | 0,20 - 10,00 |
| Nonionic surface-active substance | 1,00 - 30,00 |
| Antioxidant | 0,05 - 4,00 |
| Preservative | 0,05 - 1,00 |
| Liposoluble emulsion stabilizer | 0,05 - 2,00 |
| Water soluble emulsion stabilizer | 0,05 - 1,00 |
| Water soluble cellulose derivatives | 0,05 - 3,00 |
| Water | rest |

The following method of preparation of the composition is proposed:
- the specified amounts of nonionic surface-active substance and antioxidant are mixed and heated to the temperature in the range 40 - 120°C;
- the required amount of liposoluble emulsion stabilizer and 6-decaprenyl-2,3-dimethoxy-5-methyl-1,4-benzoquinone are dissolved in the resulting solution;
- the obtained mixture is cooled to the temperature in the range 30 - 60°C;
- this mixture is added by intensive mixing to the preheated to 30 - 60°C mixed water solution of water soluble cellulose derivatives, water soluble emulsion stabilizer and preservative.

The active substance in the proposed composition is 6-decaprenyl-2,3-dimethoxy-5-methyl-1,4-benzoquinone (ubiquinone) - the coenzyme, that plays an important role in cell bioenergetics of most prokaryotes and all eucaryotes . The main function of this substance is electron and proton transport from different substrates to cytochromes during cellular respiration and oxidative phosphorylation.

The other function of this substance is antioxidant protection of low-density lipoproteins and cell membranes from damaging actions of free radicals. These two functions are important for prophylaxis and therapy of different diseases and/or restoration of efficiency.

If the content of 6-decaprenyl-2,3-dimethoxy-5-methyl-1,4-benzoquinone in a composition is more than 10,00 wt.%, the resulting system is instable. In 2-3 weeks of storage the complex precipitate deposits in the composition that does not allow to use the proposed composition as a therapeutic agent. Reduction of ubiquinone content less than 0,20 wt.% is inappropriate because such composition is highly diluted that limits its efficiency.

A nonionic surface-active substance in the proposed composition is required to dissolve the active substance - is 6-decaprenyl-2,3-dimethoxy-5-methyl-1,4-benzoquinone and used as an emulsifier for stabilization of water emulsion. Cremophor EL (BASF), Cremophor RH - 40 (BASF), Macrogol-15-hydroxystearate, Tween-20 (Polysorbate 20), Tween-60 (Polysorbate 60), Tween-80 (Polysorbate 80), their analogues as well as their mixtures can be used as a nonionic surface-active substance in this composition.

Cremophor EL is a nonionic surface-active substance, prepared by reacting 30-35 moles of ethylene oxide with 1 mole of castor oil (polyethoxylated castor oil, or 3-polyoxyethylene glycerol ricinoleate, or glycerol polyethylene glycol ricinoleate).

Cremophor RH - 40 is a nonionic surface-active substance, prepared by reacting 40 moles of ethylene oxide with 1 mole hydrogenated castor oil (polyoxil-40-hydrogenated castor oil, or castor oil polyoxil hydrogenated).

Macrogol-15-hydroxystearate (Solutol HS 15) is a nonionic surface-active substance, prepared by reacting 15 moles of ethylene oxide with 1 mole 12-hydroxystearic acid.

Tween-20, Tween-60 and Tween-80 are nonionic surface-active substances, prepared by reacting sorbitans (esters of anhydrosorbit and fatty acids) with ethylene oxide. The main components of Tweens are ethoxylated pentaerythritol esters of lauric, stearic and oleic acids (rate of etherification 1,50 -1,65 and rate of oxyethylation 20).

If the content of nonionic surface-active substances in a composition is more than 30 wt.%, the resulting system is instable: the composition segregates, i.e. the oil and water phase separate. It does not allow to use the proposed composition. Reduction of nonionic surface-active substances content to concentrations less than 1 wt.% leads to deposition of a complex precipitate in the composition and subsequent segregation of the composition. It also does not allow to use the proposed composition.

An antioxidant in the proposed composition is required to prevent oxidation of the active substance. Alfa-tocopherol, alfa-tocopherol acetate and alfa-tocopherol succinate can be used as an antioxidant in this composition. It is possible to use other antioxidants such as butylhydroxytoluene, butylhydroxyanisole and others, as well as their mixtures.

If the content of antioxidant is more than 4,00 wt.%, the composition is instable. It segregates that makes it unusable. Reduction of the antioxidant amount to concentrations less than 0,05 wt.% leads to oxidation of the active substance- 6-decaprenyl-2,3-dimethoxy-5-methyl-1,4-benzoquinone within 2-3 weeks, that makes the composition unusable.

Preservative is required to prevent the growth of microorganisms in the proposed composition during storage. Sorbic, cinnamic and benzoic acids or sodium or potassium salts of these acids, other substances with similar properties as well as their mixtures can be used as a preservative.

If the content of preservative is more than 1 wt.%, in 2-3 weeks a white flocculent precipitate deposits in the composition, that makes it unusable. The preservative amount in concentrations less than 0,05 wt.% is not enough to prevent the growth of microorganisms in the composition during storage. As the result in 2-3 months of storage the composition does not meet the requirement of microbiological purity that makes it unusable.

Liposoluble emulsion stabilizer in the proposed composition is required for stabilization of the emulsion. This component is in the oil phase. Ascorbyl stearate, ascorbyl palmitate, sorbitan trioleate, sorbitan monolaurate, sorbitan-monopalmitate and its analogues, as well as their mixtures can be used as a liposoluble emulsion stabilizer.

If the content of a liposoluble emulsion stabilizer is more than 2,00 wt.%, the composition separates. It almost immediately precipitates that makes the composition unusable. Reduction of a liposoluble emulsion stabilizer to concentrations less than 0,05 wt.% makes the emulsion instable. There is turbidity of the composition due to agglomeration of particles in the oil phase and in 2-3 weeks the resulting product separates that makes the composition unusable.

Water soluble cellulose derivatives in the proposed composition are required to increase the emulsion stabilization. This component is in the water phase.

Methylcellulose, ethylcellulose, hydroxyethyl cellulose, hydroxypropyl cellulose and its analogues as well as their mixtures can be used as water soluble cellulose derivatives.

If the content of water soluble cellulose derivatives is more than 3,00 wt.%, the composition is very viscous. That causes serious problems with dosing of the composition. Reduction of water soluble cellulose derivatives to concentrations less than 0,05 wt.% decreases stability of the resulting composition to the stability known from the prior art of the composition.

Ethylene diamine tetraacetic, citric, tartaric or succinic acid, sodium or potassium salts of these acids, or its analogues as well as its mixtures is used as a water soluble emulsion stabilizer.

If the content of a water soluble emulsion stabilizer is more 1,00 wt.% the composition separates. It almost immediately precipitates that makes the composition unusable. Reducing the amount of a water soluble emulsion stabilizer to concentrations less than 0,05 wt.% the resulting emulsion also separates.

The preparation method of the proposed composition is the following: the specified amounts of nonionic surface-active substance and antioxidant are mixed and heated to 40 - 120°C, the required amounts of liposoluble emulsion stabilizer and 6-decaprenyl-2,3-dimethoxy-5-methyl-1,4-benzoquinone are dissolved in the resulting solution.

Chemical analyses show that dissolving the active substance at temperatures above 120°C 6-decaprenyl-2,3-dimethoxy-5-methyl-1,4-benzoquinone oxidizes fast that makes the proposed composition unusable. Dissolving the active substance at temperatures below 40°C slows down the process of obtaining a solution of liposoluble components. That leads to oxidation of 6-decaprenyl-2,3-dimethoxy-5-methyl-1,4-benzoquinone, and as the result makes the composition unusable.

Then the obtained mixture is cooled to 30 - 60°C and added by intensive mixing to preheated to 30 - 60°C mixed water solution of water soluble cellulose derivatives, water soluble emulsion stabilizer and preservative.

Heating water solution of water soluble cellulose derivatives, water soluble emulsion stabilizer and preservative to temperatures above 60°C dramatically decreases solubility of water soluble cellulose derivatives up to it full precipitation and as the result the proposed composition separates. If the temperature of water solution of water soluble cellulose derivatives, water soluble emulsion stabilizer and preservative is below 30°C, the resulting emulsion is instable that leads to separation of the proposed composition and makes it unusable.

The obtained water-oil emulsion is poured into sterile vials of dark glass and sealed. The composition is stored at the room temperature in a dark place.

Biological experiments were carried out by the known methodology on male rabbits weighted 2,0-2,5 kg. To obtain reliable results at least 5 rabbits were used in parallel series of experiments. The time of appearance of 6-decaprenyl-2,3-dimethoxy-5-methyl-1,4-benzoquinone (ubiquinone) in blood was determined in vivo after oral introduction of the composition, followed by plasma sampling from an auricle. The amount of the active substance introduced was 0,03 g in all cases.

To the known compositions [RF Nº 2008128728] (in a form of powder) and [RF Nº 2290176] the mean appearance time of ubiquinone in blood of rabbits is 19 ± 4 min and 20 ± 4 min, respectively.

Table 1 shows that the time of appearance of 6-decaprenyl-2,3-dimethoxy-5-methyl-1,4-benzoquinone in blood of rabbits for the proposed compositions is analogous to the known compositions.

The feasibility of the proposed invention is showed by the following examples, but not limited to them.

### Example 1.

10,0 g of a nonionic surface-active substance Macrogol-15-hydroxystearat and 0,5 g of an antioxidant - butylhydroxytoluene are mixed, heated to 80°C, 0,5 g of a liposoluble emulsion stabilizer - ascorbyl stearate and 2,0 g of 6-decaprenyl-2,3-dimethoxy-5-methyl-1,4-benzoquinone are dissolved in the resulting solution. Then the solution is cooled to 60°C. 0,5 g of a water soluble cellulose derivative - hydroxypropyl cellulose, 0,5 g of a preservative - cinnamic acid and 0,5 g of a water soluble emulsion stabilizer - tartaric acid are dissolved in 985,5 g of distilled water. The prepared water solution is heated to 60°C and the obtained solution of liposoluble components in the nonionic surface-active substance is added to it by intensive mixing.

The resulting water-oil emulsion is poured into 20 ml vials of dark glass, sealed and kept in a dark place. Periodically in 6 months 3 vials are sampled from this series and analyzed by chemical, physical-chemical and microbial methods.

According to chemical and physical-chemical analyses the concentration of components, the emulsion pH, its appearance and density remain unchanged within the measurement error (5-10 %) during the observation period of 36 months.

According to microbial analysis the studied composition is microbiologically pure.

Biological experiments on rabbits showed that for this composition the time of appearance of ubiquinone in blood of rabbits is 19 ± 4 min.

### Example 2.

50,0 g of a nonionic surface-active substance Tween-20 and 1,0 g of an antioxidant - alpha-tocopherol are mixed, heated to 40°C, 5,0 g of a liposoluble emulsion stabilizer - sorbitan trioleate and 10,0 g of 6-decaprenyl-2,3-dimethoxy-5-methyl-1,4-benzoquinone are dissolved in the resulting solution. Then the solution is cooled to 30°C. 5,0 g of a water soluble cellulose derivative - hydroxyethyl cellulose, 10,0 g of a preservative - sorbic acid and 3,0 g of a water soluble emulsion stabilizer - disodium EDTA are dissolved in 916,0 g of distilled water. The prepared water solution is heated to 30°C and the obtained solution of liposoluble components in the nonionic surface-active substance is added to it by intensive mixing.

The resulting water-oil emulsion is poured into 20 ml vials of dark glass, sealed and kept in a dark place. Periodically in 6 months 3 vials are sampled from this series and analyzed by chemical, physical-chemical and microbial methods.

According to chemical and physical-chemical analyses the concentration of components, the emulsion pH, its appearance and density remain unchanged within the measurement error (5-10 %) during the observation period of 36 months.

According to microbial analysis the studied composition is microbiologically pure.

Biological experiments on rabbits showed that for this composition the time of appearance of ubiquinone in blood of rabbits is 17 ± 5 min.

### Example 3.

200,0 g of a nonionic surface-active substance Cremophor EL and 40,0 g of an antioxidant - alfa-tocopherol succinate are mixed, heated to 120°C, 20,0 g of a liposoluble emulsion stabilizer - sorbitan monolaurate and 60,0 g of 6-decaprenyl-2,3-dimethoxy-5-methyl-1,4-benzoquinone are dissolved in the resulting solution. Then the solution is cooled to 40°C. 10,0 g of a water soluble cellulose derivative - ethyl cellulose, 10,0 g of a preservative - potassium sorbate and 8,0 g of a water soluble emulsion stabilizer - citric acid are dissolved in 652,0 g of distilled water. The prepared water solution is heated to 50°C and the obtained solution of liposoluble components in the nonionic surface-active substance is added to it by intensive mixing.

The resulting water-oil emulsion is poured into 20 ml vials of dark glass, sealed and kept in a dark place. Periodically in 6 months 3 vials are sampled from this series and analyzed by chemical, physical-chemical and microbial methods.

According to chemical and physical-chemical analyses the concentration of components, the emulsion pH, its appearance and density remain unchanged within the measurement error (5-10 %) during the observation period of 36 months.

According to microbial analysis the studied composition is microbiologically pure.

Biological experiments on rabbits showed that for this composition the time of appearance of 6-decaprenyl-2,3-dimethoxy-5-methyl-1,4-benzoquinone in blood of rabbits is 20 ± 4 min.

### Example 4.

100,0 g of a nonionic surface-active substance Tween-60, 200,0 g of a nonionic surface-active substance Tween-80, 20,0 g of an antioxidant - alpha-tocopherol acetate are mixed, heated to 100°C, 10,0 g of a liposoluble emulsion stabilizer - ascorbyl palmitate and 100,0 g of 6-decaprenyl-2,3-dimethoxy-5-methyl-1,4-benzoquinone are dissolved in the resulting solution. Then the solution is cooled to 40°C. 30,0 g a water soluble cellulose derivative - methyl cellulose, 1,0 g of a preservative- sodium benzoate and 5,0 g of a water soluble emulsion stabilizer - ethylenediaminetetraacetic acid are dissolved in 534,0 g of distilled water. The prepared water solution is heated to 60°C and the obtained solution of liposoluble components in the nonionic surface-active substance is added to it by intensive mixing.

The resulting water-oil emulsion is poured into 20 ml vials of dark glass, sealed and kept in a dark place. Periodically in 6 months 3 vials are sampled from this series and analyzed by chemical, physical-chemical and microbial methods.

According to chemical and physical-chemical analyses the concentration of components, the emulsion pH, its appearance and density remain unchanged within the measurement error (5-10 %) during the observation period of 36 months.

According to microbial analysis the studied composition is microbiologically pure.

Biological experiments on rabbits showed that for this composition the time of appearance of 6-decaprenyl-2,3-dimethoxy-5-methyl-1,4-benzoquinone in blood of rabbits is 19 ± 5 min.

### Example 5.

250,0 g of a nonionic surface-active substance Cremophor RH-40, 5,0 g of an antioxidant- alpha-tocopherol acetate and 5,0 g of an antioxidant- alfa-tocopherol are mixed, heated to 60°C, 5,0 g of a liposoluble emulsion stabilizer- sorbitan monolaurate, 5,0 g of a liposoluble emulsion stabilizer- ascorbyl palmitate and 70,0 g of 6-decaprenyl-2,3-dimethoxy-5-methyl-1,4-benzoquinone are dissolved in the resulting solution. Then the solution is cooled to 45°C. 10,0 g a water soluble cellulose derivative - methyl cellulose, 15,0 g a water soluble cellulose derivative - hydroxypropylcellulose, 2,0 g of a preservative - benzoic acid, 4,0 g of a preservative-cinnamic acid, 0,5 g a water soluble emulsion stabilizer - succinic acid and 0,5 g a water soluble emulsion stabilizer - citric acid are dissolved in 628,0 g of distilled water. The prepared water solution is heated to 50°C and the obtained solution of liposoluble components in the nonionic surface-active substance is added to it by intensive mixing.

The resulting water-oil emulsion is poured into 20 ml vials of dark glass, sealed and kept in a dark place. Periodically in 6 months 3 vials are sampled from this series and analyzed by chemical, physical-chemical and microbial methods.

According to chemical and physical-chemical analyses the concentration of components, the emulsion pH, its appearance and density remain unchanged within the measurement error (5-10 %) during the observation period of 36 months.

According to microbial analysis the studied composition is microbiologically pure.

Biological experiments on rabbits showed that for this composition the time of appearance of 6-decaprenyl-2,3-dimethoxy-5-methyl-1,4-benzoquinone in blood of rabbits is 18 ± 4 min.

Thus the proposed composition is a water-oil emulsion, stable during 36 months, with high bioavailability and prolonged shelf life.

**Table 1**

| Composition | The time of appearance of ubiquinone in blood of rabbits (min) |
|---|---|
| The known composition [RF Nº 2008128728] (in a form of powder) | 19 ± 4 |
| The known composition [RF Nº 2290176] | 19 ± 4 |
| Example 1 | 19 ± 4 |
| Example 2 | 17 ± 5 |
| Example 3 | 20 ± 4 |
| Example 4 | 19 ± 5 |
| Example 5 | 18 ± 4 |

## Claims

1. A composition based on 6-decaprenyl-2,3-dimethoxy-5-methyl-1,4-benzoquinone, comprising the active substance - 6-decaprenyl-2,3-dimethoxy-5-methyl-1,4-benzoquinone, a nonionic surface-active substance, an antioxidant, a preservative, a liposoluble emulsion stabilizer, a water soluble emulsion stabilizer, and water, wherein the composition further comprises water soluble cellulose derivatives with the following proportion of components, wt.%:
| | |
|---|---|
| 6-decaprenyl-2,3-dimethoxy-5-methyl-1,4-benzoquinone | 0,20 - 10,00 |
| Nonionic surface-active substance | 1,00 - 30,00 |
| Antioxidant | 0,05 - 4,00 |
| Preservative | 0,05 - 1,00 |
| Liposoluble emulsion stabilizer | 0,05 - 2,00 |
| Water soluble emulsion stabilizer | 0,05 - 1,00 |
| Water soluble cellulose derivatives | 0,05 - 3,00 |
| Water | rest |

2. The composition according to claim 1, **characterized by** the use of Cremophor EL, Cremophor RH-40, macrogol-15-hydroxystearat, Tween-20, Tween-60, Tween - 80 or their mixtures as nonionic surface-active substance.

3. The composition according to claim 1, **characterized by** the use of alpha-tocopherol, alpha-tocopherol acetate, alpha-tocopherol succinate, buthylhydroxytoluene, butyl hydroxyanisol or their mixtures as antioxidant.

4. The composition according to claim 1, **characterized by** the use of sorbic, cinnamic and benzoic acids, sodium or potassium salts of these acids or their mixtures as preservative.

5. The composition according to claim 1, **characterizing by** the use of ascorbyl stearate, ascorbyl palmitate, sorbitan trioleate, sorbitan monolaurate, sorbitan monopalmitate or their mixtures as liposoluble emulsion stabilizer.

6. The composition according to claim 1, **characterizing by** the use of methyl cellulose, ethyl cellulose, hydroxyethyl cellulose, hydroxypropyl cellulose or their mixtures as water soluble cellulose derivatives.

7. The composition according to claim 1, **characterizing by** the use ethylenediaminotetraacetic acid, citric acid, tartaric acid, succinic acid, sodium or potassium salts of these acids or their mixtures as water soluble emulsion stabilizer.

8. The preparation method of the pharmaceutical composition based on 6-decaprenyl-2,3-dimethoxy-5-methyl-1,4-benzoquinone is the following:
nonionic surface-active substance and antioxidant are mixed and heated to the temperature in the range 40 - 120°C;
liposoluble emulsion stabilizer and 6-decaprenyl-2,3-dimethoxy-5-methyl-1,4-benzoquinone are dissolved in the resulting solution; the obtained mixture is cooled to the temperature in the range 30 - 60°C;
this mixture is added by intensive mixing to the preheated to 30 - 60°C mixed water solution of water soluble cellulose derivatives, water soluble emulsion stabilizer and preservative.
